# EUROPEAN PATENT APPLICATION

(11) **EP 2 168 945 A1**
(43) Date of publication of application: **31.03.2010**
(21) Application number: 08016965.9
(22) Date of filing: 26.09.2008
(51) Int. Cl.: C07C 229/48

(54) **Crystalline forms of Tilidine Mesylate**

(71) Applicant: Hexal AG, 83607 Holzkirchen (DE)
(72) Inventor: Born, Max, 83627 Warngau (DE)
(74) Representative: Maiwald Patentanwalts GmbH

(57) **Abstract**

The present invention provides novel crystalline forms of Tilidine Mesylate designated as forms I and II and processes for their preparation. The form I is a hygroscopic and crystalline Tilidine Mesylate, whereas the form II is not hygroscopic, water containing and crystalline.

## Description

The present invention relates to crystalline forms of the mesylate salt of Tilidine, processes for their preparation and their use in pharmaceutical compositions.

(±)-Ethyl-*trans*-2-(dimethylamino)-1-phenyl-3-cyclohexene-1-carboxylate (Tilidine) is a pharmaceutically active ingredient with strong analgesic properties. Tilidine is a base known to have a very low melting point, which may create problems for the manufacture of pharmaceutical compositions. Therefore, Tilidine is generally used in pharmaceutical compositions in the form of the hydrochloride hemihydrate salt which is more stable than the free base. The hydrochloride hemihydrate salt of Tilidine can easily be prepared into solutions and certain types of soft capsules. However, there may arise stability problems if the hydrochloride hemihydrate of Tilidine is contained in solid formulations such as tablets and the like.

In order to overcome the known stability problems encountered when preparing solid pharmaceutical compositions containing Tilidine salts, in particular the hydrochloride hemihydrate thereof, the use of salts having improved stability properties such as the orthophosphate or mesylate salt of Tilidine has been suggested in the state of the art.

EP 0 665 830 describes a Tilidine dihydrogen orthophosphate, which is said to be stable and therefore suitable for the preparation of solid pharmaceutical formulations such as tablets or dragees. However, the preparation of the orthophosphate appears to be difficult and challenging to the practitioner as to the process conditions which have to be closely complied with.

EP 1 073 625 describes a Tilidine Mesylate salt having improved stability compared to the hydrochloride hemihydrate of Tilidine. The mesylate salt disclosed in EP 1 073 625 is according to the reported elemental analysis a crystalline and water free salt, i.e. an anhydrate. Said form has a melting point of 136 °C, and it is not hygroscopic. Moreover, the Tilidine Mesylate of EP 1 073 625 shows excellent solubility in water giving almost neutral and stable solutions. Aforementioned properties of Tilidine Mesylate render it suitable for the manufacture of granules, tablets, dragees or pills, i.e. solid pharmaceutical compositions.

EP 1 140 031 discloses sustained release pharmaceutical compositions containing Tilidine Mesylate as an active ingredient. In particular, solid pharmaceutical compositions for oral administration having a controlled-release matrix containing Tilidine Mesylate, wherein the controlled-release matrix comprises a gel forming matrix material.

It is commonly known in the art that pharmaceutically active substances have to meet strict requirements regarding chemical and physical properties in order to enable formulations which comply with the pharmaceutical requirements and specifications. For example, active substances which differ in the solid state often show differences in the solubility and thus bioavailability as well. In particular, differences in the chemical and physical properties may exist between the amorphous and crystalline state of a substance, but also between different polymorphic forms and/or pseudopolymorphic forms (hydrates).

The present inventors have now surprisingly and unexpectedly found that Tilidine Mesylate may exist in different crystalline forms. Hence, the present invention provides Tilidine Mesylate in two different crystalline forms, on the one hand, a hygroscopic crystalline form, which is a water free form (anhydrate), and on the other hand, a non-hygroscopic, water containing crystalline form (e.g., a dihydrate) of Tilidine Mesylate.

Accordingly, the present invention relates to a hygroscopic crystalline form of Tilidine Mesylate.

Preferably, the hygroscopic crystalline form of Tilidine Mesylate is one having an X-ray powder diffraction pattern containing the following 2θ values: 9.46, 16.03 and 18,97 ± 0.2° (hereinafter the form I).

The x-ray powder diffraction pattern of the form I further contains the following 2θ values: 13.07, 13.82, 14.90, 19.02, 20.34 and 25.22 ± 0.2°.

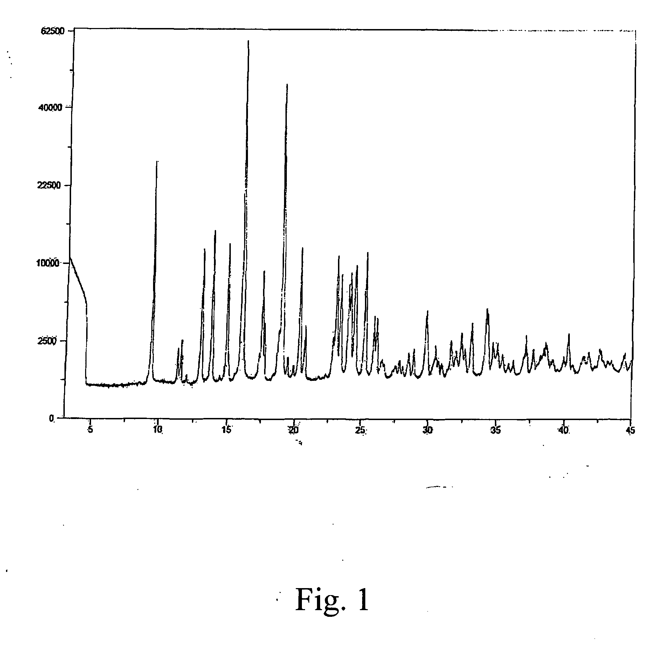

The complete X-ray powder diffraction pattern of the form I is depicted in Fig. 1.

The hygroscopic crystalline form I of Tilidine Mesylate is considered to represent the high temperature modification of Tilidine Mesylate due to its relatively high melting point of about 140 °C as determined by DSC, which is higher than the 136 °C reported for the anhydrate form disclosed in EP 1 073 625 (as confirmed by the present inventors by DSC measurement). Moreover, contrary to what has been reported for the anhydrate of EP 1 073 625, the water free crystalline form of Tilidine Mesylate according to the present invention is hygroscopic.

The hygroscopic crystalline form of Tilidine Mesylate according to the present invention can be prepared as described in example 1 of EP 1 073 625. The process comprises the steps of (a) dissolving Tilidine base in a suitable solvent such as ethyl acetate, (b) adding an equimolar amount of mesylic acid, (c) precipitating Tilidine Mesylate optionally by cooling the solution, and (d) separating and drying the precipitated Tilidine Mesylate.

In examples 1 to 3 of EP 1 073 625 the non-hygroscopic Tilidine Mesylate anhydrate was obtained by drying the precipitate under vacuum. It has been surprisingly and unexpectedly found that by drying the precipitated product by heating it, e.g. in a drying closet, at ambient pressure, i.e. without applying a vacuum, a hygroscopic crystalline Tilidine Mesylate, such as the form I, is obtained. In particular, the hygroscopic crystalline form of Tilidine Mesylate according to the present invention is obtained after heating the precipitate at ambient pressure and at about 100 °C for at least one hour, preferably about two to three hours.

The form I of the Tilidine Mesylate according to the present invention is chemically and physically stable and thus suitable in the manufacture of pharmaceutical compositions.

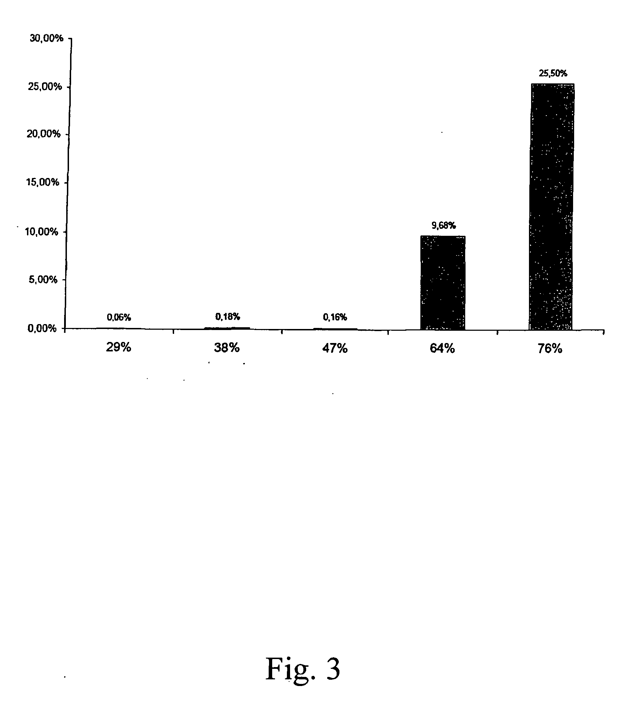

Regarding the hygroscopicity observed for the form I it has to be pointed out that no water uptake of the form I could be observed at a relative humidity of the environment below about 50% as depicted in Fig. 3. Fig. 3 shows the increase of the mass of the form I after having stored the form I for 22.5 hours at the specified relative humidities of 29%, 38%, 47%, 64% and 76%. It was confirmed by XRD measurement that no changes occurred in the crystal structure of form I when stored at 29%, 38% or 47% relative humidity.

In a further aspect, the present invention provides a non-hygroscopic, water containing crystalline form of Tilidine Mesylate, such as a dihydrate.

It has been surprisingly found that there exists a non-hygroscopic, water containing crystalline form of Tilidine Mesylate which is a dihydrate having a well defined crystal structure, as confirmed by an XRD measurement, and which is designated as form II.

The form II has an X-ray powder diffraction pattern which is characterized by the following 2θ values: 12.25, 12.89, 17.34 and 21.58 ± 0.2°.

Moreover, the form II XRD pattern further contains the following 2θ values: 6.44, 11.27, 20.36, 21.32, 21.73, 21.93, 24.12, 27.92 and 28.59 ± 0.2°.

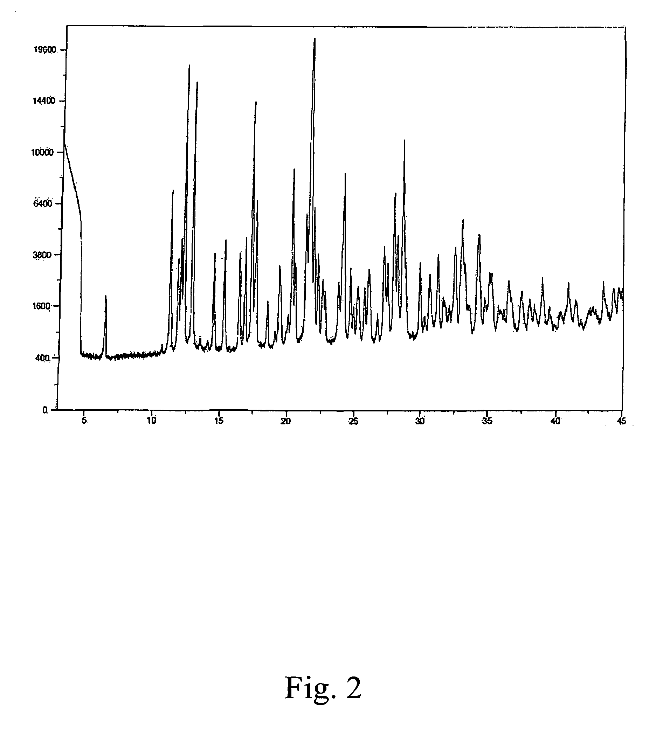

The complete X-ray powder diffraction pattern of the form II is depicted in Fig. 2.

As determined by DSC, the non-hygroscopic, water containing crystalline form of Tilidine Mesylate according to the present invention has a melting point of about 61 °C, which form is therefore be considered to represent a low temperature modification of Tilidine Mesylate compared to the form II of the present invention or that form disclosed in EP 1 073 625.

The non-hygroscopicity of form II was determined by the method described in European pharmacopoeia 6.2 / 5.2. Characters section in monographs "Hygroscopicity"

In a further aspect, the present invention relates to the use of a hygroscopic crystalline form of Tilidine Mesylate in the preparation of a non-hygroscopic, water containing crystalline form of Tilidine Mesylate.

The inventors have surprisingly found that the hygroscopic crystalline form of Tilidine Mesylate, in particular the form I, is suitable for the preparation of a non-hygroscopic, water containing crystalline form of Tilidine Mesylate such as the form II.

The hygroscopic crystalline form of Tilidine Mesylate according to the present invention appears to uptake of water under alteration of the crystalline state. For example, the form II was obtained after having stored the form I levigated with form II of the present invention in a humid atmosphere, preferably in an environment having a relative humidity of about 64% for 24 hours.

Therefore, the present invention also relates to a non-hygroscopic, water containing crystalline form of Tilidine Mesylate, such as the form II, obtainable by exposing the hygroscopic crystalline form of Tilidine Mesylate, in particular form I together with seeding crystals form II, to a humid atmosphere.

In a further aspect, the present invention also relates to pharmaceutical compositions containing aforementioned crystalline forms of Tilidine Mesylate of the present invention. Preferably, the pharmaceutical composition of the present invention is a solid formulation for oral administration containing the form I or II of Tilidine Mesylate.

The peak listings for the X-ray powder diffraction pattern (see Figures 1 and 2) obtained for the forms I and II of Tilidine Mesylate are given below.

| **Form I** | |
|---|---|
| Pos. [°2θ] | Rel. Int. [%] |
| 4,42 | 4,67 |
| 4,47 | 2,62 |
| 8,58 | 0,17 |
| 9,46 | 45,65 |
| 11,41 | 2,60 |
| 11,63 | 3,70 |
| 12,01 | 0,60 |
| 13,07 | 19,41 |
| 13,48 | 0,38 |
| 13,82 | 24,01 |
| 14,46 | 0,54 |
| 14,93 | 20,47 |
| 15,06 | 1,77 |
| 16,03 | 100,00 |
| 16,08 | 40,35 |
| 17,28 | 2,24 |
| 17,49 | 14,98 |
| 17,68 | 5,52 |
| 18,71 | 4,80 |
| 18,97 | 79,05 |
| 19,02 | 43,70 |
| 19,43 | 2,02 |
| 19,90 | 1,33 |
| 20,34 | 19,88 |
| 20,74 | 5,19 |
| 21,80 | 0,54 |
| 22,30 | 0,64 |
| 22,81 | 3,93 |
| 23,07 | 17,45 |
| 23,38 | 14,14 |
| 23,45 | 5,95 |
| 23,95 | 12,22 |
| 24,11 | 14,38 |
| 24,18 | 7,08 |
| 24,41 | 14,19 |
| 24,49 | 15,81 |
| 25,22 | 18,92 |
| 25,30 | 7,63 |
| 25,86 | 6,76 |
| 25,93 | 3,90 |
| 26,08 | 6,38 |
| 26,15 | 2,98 |
| 26,41 | 1,53 |
| 26,51 | 1,76 |
| 26,68 | 1,39 |
| 27,54 | 1,22 |
| 27,85 | 1,64 |
| 28,07 | 1,18 |
| 28,50 | 2,32 |
| 28,90 | 2,61 |
| 29,81 | 7,52 |
| 29,89 | 4,22 |
| 30,47 | 3,01 |
| 30,57 | 1,84 |
| 30,69 | 1,62 |
| 30,94 | 1,34 |

| **Form II** | |
|---|---|
| Pos. [°2θ] | Rel. Int. [%] |
| 4,48 | 5,53 |
| 6,44 | 9,81 |
| 10,64 | 0,98 |
| 11,27 | 32,25 |
| 11,82 | 14,44 |
| 12,02 | 22,53 |
| 12,25 | 80,79 |
| 12,89 | 96,99 |
| 13,46 | 1,59 |
| 14,01 | 1,25 |
| 14,51 | 15,66 |
| 15,29 | 18,52 |
| 16,43 | 18,62 |
| 16,84 | 19,82 |
| 17,34 | 73,18 |
| 17,64 | 33,80 |
| 18,50 | 7,27 |
| 19,09 | 2,57 |
| 19,38 | 15,26 |
| 19,44 | 14,21 |
| 19,52 | 7,28 |
| 20,04 | 4,52 |
| 20,36 | 41,93 |
| 20,57 | 13,32 |
| 21,32 | 26,36 |
| 21,38 | 22,17 |
| 21,58 | 92,51 |
| 21,66 | 100,00 |
| 21,73 | 38,74 |
| 21,93 | 29,44 |
| 22,24 | 15,31 |
| 22,58 | 12,05 |
| 22,76 | 8,07 |
| 23,77 | 10,36 |
| 23,99 | 16,71 |
| 24,12 | 41,31 |
| 24,19 | 20,33 |
| 24,65 | 11,74 |
| 24,85 | 6,10 |
| 25,16 | 8,51 |
| 25,22 | 10,13 |
| 25,29 | 6,86 |
| 25,70 | 9,29 |
| 25,94 | 11,83 |
| 26,02 | 12,61 |
| 26,11 | 9,54 |
| 26,62 | 4,66 |
| 27,15 | 17,85 |
| 27,22 | 13,06 |
| 27,41 | 14,17 |
| 27,92 | 35,66 |
| 27,99 | 18,07 |
| 28,17 | 20,01 |
| 28,59 | 52,16 |
| 28,67 | 26,93 |
| 28,77 | 16,25 |
| 28,85 | 9,33 |
| 29,89 | 13,72 |
| 29,98 | 6,35 |
| 30,22 | 4,22 |
| 30,61 | 10,86 |
| 30,69 | 8,93 |

### Examples

### Example 1: Preparation of Tilidine Mesylate form I

30 g (0.01 mol) of Tilidine base were dissolved in 50 ml of ethyl acetate at 40 °C under stirrig. Subsequently, 10.5 g (0.01 mol) of mesylic acid were added at the same temperature. The mixture was cooled to room temperature to precipitate the product. The product was filtered off, washed with ethyl acetate and dried in a drying closet at ambient pressure and 100 °C for two hours. The material obtained was hygroscopic, and it is therefore recommended to store it in an exsiccator over silica gel.

### Example 2: Preparation of seeding crystals Form II

One Gram of Tilidine Mesylat Form I was exposed to an atmosphere having a relative humidity of about 64%. The atmosphere was established by using a closed exsiccator, in which a saturated aqueous sodium nitrite solution was in. After mass change of 8%, after about 17 hours the substance was taken out of the exsiccator and stored for 48 hours in an exsiccator filled with saturated aqueous potassium thiocyanate (46% rF) solution. Afterwards, the substance was exposed for 24 hours to an atmosphere having a relative humidity of about 76%. The atmosphere was established by using a closed exsiccator with saturated aqueous sodium chloride solution.

### Example 3: Preparation of Tilidine Mesylate form II

One Gram of Tilidine Mesylat Form I was mixed and levigated with 0.2 g seeding crystals of form II. The 1.2 g were exposed in an opened pot to an atmosphere having a relative humidity of about 64%. The atmosphere was established by using a closed exsiccator, in which the mixture was kept in an open pot for 24 hours over a saturated aqueous sodium nitrite solution.

## Claims

1. Hygroscopic crystalline form of Tilidine Mesylate.

2. A Tilidine Mesylate according to claim 1, which is the Form I, having an X-ray powder diffraction pattern containing the following 2θ values: 9.46, 16.03 and 18.97 ± 0.2°.

3. A Tilidine Mesylate according to claim 2, wherein the X-ray powder diffraction pattern further contains the following 2θ values: 13.07, 13.82, 14.90, 19.02, 20.34 and 25.22 ± 0.2°.

4. A Tilidine Mesylate according to claim 3 having the X-ray powder diffraction pattern depicted in Fig. 1.

5. A Tilidine Mesylate according to any one of the preceding claims having a melting point of about 140 °C as determined by DSC.

6. Non-hygroscopic, water containing crystalline form of Tilidine Mesylate.

7. A Tilidine Mesylate according to claim 6, which is the Form II, having an X-ray powder diffraction pattern containing the following 2θ values: 12.25, 12.89, 17.34 and 21.58 ± 0.2°.

8. A Tilidine Mesylate according to claim 7, wherein the X-ray powder diffraction pattern further contains the following 2θ values: 6.44, 11.27, 20.36, 21.32, 21.73, 21.93, 24.12, 27.92 and 28.59 ± 0.2°.

9. A Tilidine Mesylate according to claim 8 having the X-ray powder diffraction pattern depicted in Fig. 2.

10. A Tilidine Mesylate according to any one of claims 6 to 9 having a melting point of about 61 °C as determined by DSC.

11. Use of a hygroscopic crystalline form of Tilidine Mesylate in the preparation of a non-hygroscopic, water containing crystalline form of Tilidine Mesylate.

12. Use according to claim 11, wherein the hygroscopic crystalline form of Tilidine Mesylate is the form I.

13. Use according to claim 11 or 12, wherein the non-hygroscopic, water containing crystalline form of Tilidine Mesylate is the form II.

14. A non-hygroscopic, water containing crystalline form of Tilidine Mesylate obtainable by exposing the hygroscopic crystalline form of Tilidine Mesylate according to any one of claims 1 to 5 to a humid atmosphere.

15. A Tilidine Mesylate according to claim 14, which is the form II.

16. Pharmaceutical composition containing a crystalline form of Tilidine Mesylate according to any one of claims 1 to 10.
